# EUROPEAN PATENT APPLICATION

(11) **EP 4 588 924 A1**
(43) Date of publication of application: **23.07.2025**
(21) Application number: 25152603.4
(22) Date of filing: 17.01.2025
(51) Int. Cl.: C07F 9/572, G01N 33/58

(54) **PHOSPHORAMIDITE COMPOUNDS AND METHODS FOR PREPARING SYNTHETIC NUCLEIC ACIDS**

(30) Priority: 17.01.2024 US 202463621604 P
(71) Applicant: Enzo Biochem, Inc., Farmingdale, NY 11735 (US)
(72) Inventor: PANDE, Praveen, Holbrook, 11741 (US); LI, Zaiguo, Little Neck, 11362 (US); BALAKRISHNAN, Subhasree, Dix Hills, 11747 (US); SURUGIHALLI, Chaitra V., West Babylon, 11704 (US); UDDIN, Mohammad Meenhaj, Bellerose, 11426 (US)
(74) Representative: Vossius & Partner Patentanwälte Rechtsanwälte mbB

(57) **Abstract**

The present disclosure concerns phosphoramidite compounds useful for nucleic acid hybridization probes, the probes containing them, and related methods of use and preparation.

## Description

### TECHNICAL FIELD

The present disclosure relates to phosphoramidite compounds, their production, and their use in molecular biology especially in the field of nucleic acid detection and sequencing. The disclosure also relates to methods of synthesizing nucleic acids, including without limitation oligonucleotides that are oligoribonucleic acids (oligo-RNAs) or oligodeoxyribonucleic acids (oligo-DNAs).

### BACKGROUND

Oligonucleotides containing various reporter groups have widespread application in molecular biology and medicine as tools for the specific detection of sequences, in DNA sequencing, and in other molecular biology methods. These labeled oligonucleotides are also useful in the research and production of pharmaceutical materials such as without limitation antisense nucleic acids, ribozymes, and RNA for RNAi-mediated control of gene expression. Non-nucleoside-based phosphoramidites that bear the 2,4-dinitrophenyl group ("DNP") are known. These labelled phosphoramidites have been used in solid-phase oligonucleotide synthesis to attach single and multiple dinitrophenyl groups to the 5' end of oligonucleotides, and have been shown to be compatible with the normal oligonucleotide synthesis cycle. Will et al., "The synthesis of oligonucleotides that contain 2,4-dinitrophenyl reporter groups," Carbohydrate Res. Vol 216 (1992), 315-322. The DNP-labelled oligonucleotides have been detected using monoclonal and polyclonal anti-dinitrophenyl antibodies. Tatulchenkov et al. disclose synthesis of phosphoramidite reagents for introduction into oligonucleotides. Russian J. Bioinorg. Chem. 2017,43,4, pp 386-396.

Analytical tests based on detection of DNP-labelled oligonucleotides with anti-DNP antibodies have been proposed. One example of a DNP labeling reagent is 1-dimethoxytrityloxy-3-O-[N-(2,4-dinitrophenyl)-3-N-aminopropyl-(triethyleneglycol)]-glyceryl-2-O-(2-cyanoethyl)-(N,N-diisopropyl)-phosphoramidite, which is a DNP phosphoramidite with a spacer that is a branched triethylene glycol (TEG): 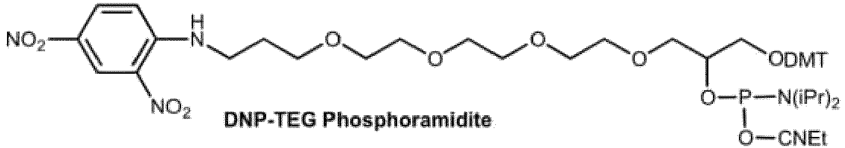 (glenresearch.com/10-1985.html accessed Dec. 17, 2023; chemgenes.com/products.php?product=CLP-9907, accessed December 17, 2023). "DMT" is dimethoxytrityl, a protecting group used often for the 5'-hydroxy group in nucleosides and nucleoside analogs particularly in oligonucleotide synthesis, having the following structure:

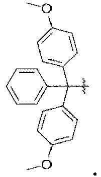

One significant problem with DNP-TEG phosphoramidite is that it may allow for an intra-molecular cyclization reaction which, to be avoided, requires a step for the removal of the cyanoethyl protection group prior to the DMT group deprotection to prevent the loss of the DNP label during synthesis via intra-molecular cyclization. It is an object of the invention to provide a DNP-labelled nucleic acid that can be prepared more efficiently and less expensively than DNP-TEG phosphoramidite nucleic acids.

US Pat. App. Pub. 20200199174A1 published June 25, 2020 discloses methods for fluorescently labeling an intracellular protein that includes obtaining a fusion protein of a protein to be labeled and an anti-DNP antibody, and then bringing a compound represented by this formula 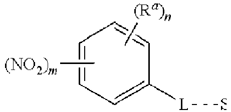 in which S is a fluorescent group, or the salt of the compound into contact with the cell, and fluorescently labeling the protein to be labeled by reacting the fusion protein and the compound or its salt. Anti-DNP antibodies and antigen-binding fragments thereof are disclosed in US Pat. App. Pub. 20200199174A1, e.g. at paras. [0056]-[0061] and are commercially available as would be known to one skilled in the art.

One application for labeled oligonucleotides is in situ hybridization, which is a method employed for the precise spatial localization and identification of distinct DNA and RNA sequences within cells, conserved tissue sections, or complete tissue samples. This technique involves the binding of a nucleic acid probe's complementary strand to a specific target sequence, thus facilitating the identification of the sought-after genetic material. These resulting hybrids are capable of providing detectable signals through various methods, dependent on the labeling strategy employed. Probes labeled with radioactive elements can be visualized utilizing autoradiography, while probes labeled with haptens can be detected through affinity-based mechanisms, wherein enzyme-mediated colorimetric signals are generated. Furthermore, for direct visualization, fluorescent markers may be attached to the probes, enabling direct detection using fluorescence, a technique commonly referred to as "fluorescent in situ hybridization" (FISH).

A significant advantage of in situ hybridization is that it facilitates the elucidation of interrelationships between the spatial distribution of precise nucleic acid sequences and the corresponding protein products of the designated gene. Moreover, this technique extends to exploring the associations between these molecular components and the underlying cellular structures. Presently, this method serves as a pivotal tool within both scientific research and clinical contexts, effectively contributing to the comprehensive understanding of diverse domains. The spatial data garnered through this approach has substantially enriched researchers' comprehension in fields such as viral infection, genetic mapping, cytogenetics, gene expression dynamics, and prenatal diagnosis and developmental processes. Methods to improve the detection of nucleic acids using in situ hybridization, such as by allowing for faster detection, detection of smaller nucleic acid sequences, detection at lower concentrations of the nucleic acid target sequence, or improved reproducibility of the detection are desired.

The present disclosure relates to novel phosphoramidite compounds, their synthesis, their use, and synthetic nucleic acids, for example synthetic oligonucleotides, containing these novel phosphoramidites. The present disclosure also provides methods for the preparation and use of synthetic nucleic acids relating to or involving the phosphoramidite compounds disclosed herein. An object of the present disclosure is to provide useful and novel phosphoramidite compounds for efficient, high-yield synthesis of labeled synthetic nucleic acids, such as without limitation oligoRNAs, for use as probes in in situ hybridization. A further object is to provide a phosphoramidite compound that can be attached to an oligoRNA probe used in nucleic acid detection and screening methods, such as in situ hybridization, wherein the phosphoramidite may be easily detected at high efficiency. The compounds of the present disclosure solve a problem of providing improved labeled oligo probes for accurate in situ hybridization and other nucleic acid sequencing and screening assays.

### SUMMARY

The present disclosure concerns a compound represented by the structure of formula (I): 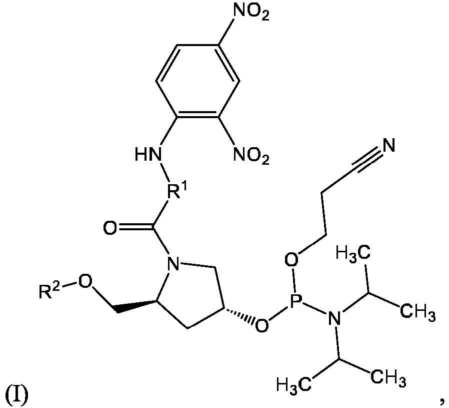
wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and -((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄ dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups and
wherein R² is C₁-C₂₀-alkyl, preferably C₁-C₆-alkyl, or any suitable protecting group for an alcohol, preferably 4,4'dimethoxytrityl.

In another embodiment, the present disclosure concerns a method of preparing a polynucleotide, the method comprising:
providing a first oligonucleotide directly or indirectly linked to a solid support at the 3'-OH of the oligonucleotide sugar moiety and having a free 5' -OH group;
activating the compound of the formula (I) or (II), providing the activated compound to the first oligonucleotide to form a phosphite triester bond to form a second oligonucleotide;
oxidizing the second polynucleotide;
deprotecting the second polynucleotide to remove R²;
optionally activating a nucleotide, non-naturally occurring nucleotide or phosphoramidite and coupling to the second polynucleotide, oxidizing the resulting third polynucleotide, and deprotecting the third polynucleotide; and
cleaving the resulting polynucleotide from the solid support.

In another embodiment, the present disclosure is directed to method of making a compound represented by the formula: 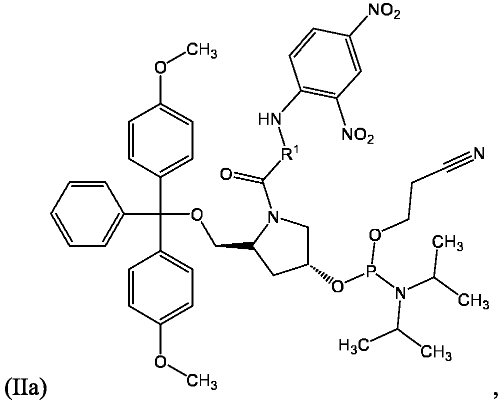 the method comprising:
contacting a compound represented by the formula: 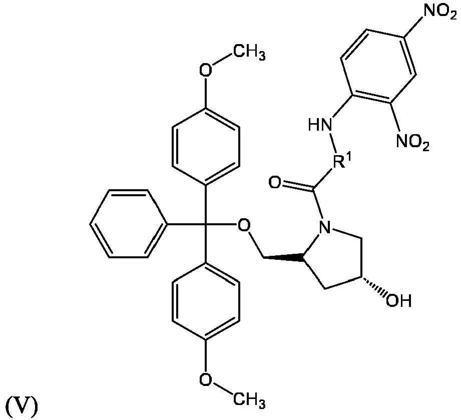 with 2-cyanoethyl N,N,N',N'-tetraisopropylphosphorodiamidite, wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and -((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the
   C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄ dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups.

In a further embodiment, the present disclosure is directed to a method of making a compound represented by the formula: 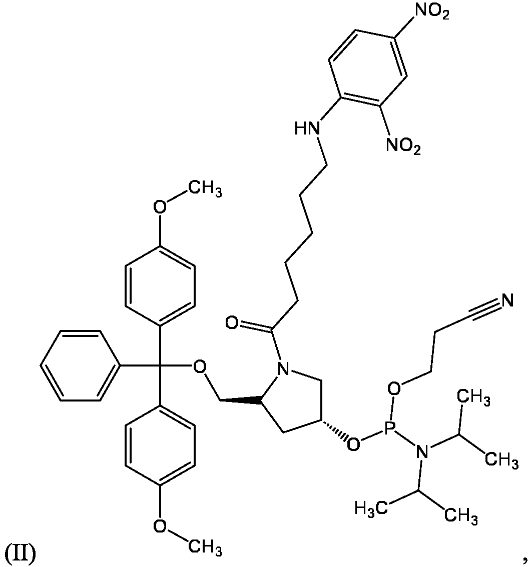 the method comprising: contacting a compound represented by the formula (VI) 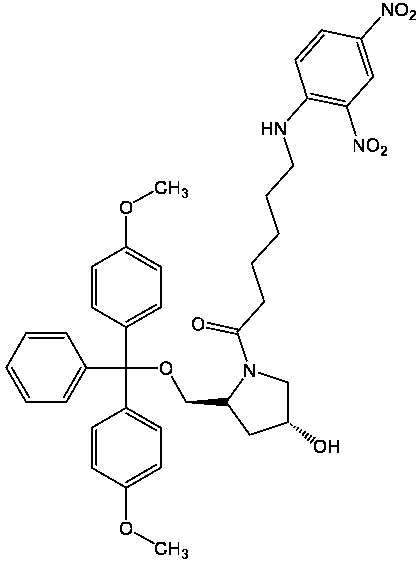 with 2-cyanoethyl N,N,N',N'-tetraisopropylphosphorodiamidite.

In a further embodiment, the disclosure concerns a polynucleotide, optionally, a nucleic acid hybridization probe, comprising the compound represented by the formula: 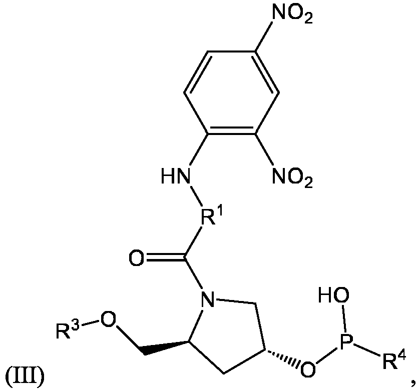
wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and -((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄ dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups and
wherein R³ and R⁴ are the same or different and R³ is selected from the group consisting of C₁-C₂₀-alkyl, preferably C₁-C₆-alkyl, or any suitable protecting group for an alcohol, preferably 4,4'dimethoxytrityl, a naturally occurring nucleotide, nucleotide analogs and phosphoramidites, and R⁴ is selected from the group consisting of a naturally occurring nucleotide, nucleotide analogs and phosphoramidites
wherein R³ and R⁴ are attached to the compound of formula (III) by phosphodiester bonds.

In another embodiment, the present disclosure is directed to polynucleotide comprising a compound represented by the formula: 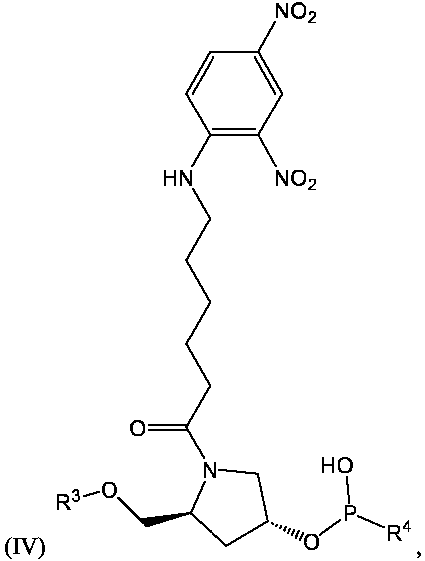
wherein R³ and R⁴ are the same or different and are a naturally occurring nucleotide, or are monomers selected from the group consisting of a second compound of formula (IV), nucleotide analogs and phosphoramidites, and
wherein R³ and R⁴ are attached to the compound of formula (III) by phosphodiester bonds.

### BRIEF DESCRIPTION OF THE DRAWINGS

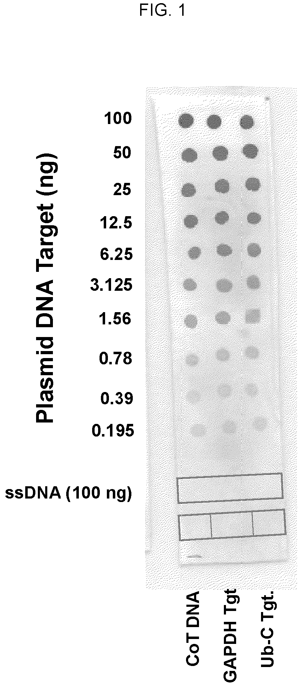

FIG. 1 shows dot-blot for the hybridization of EGFR oligonucleotide probes with various concentrations of EGFR plasmid DNA (from 100 ng to 0.195 ng input). A distinct staining intensity pattern was observed from highest to lowest concentration of target plasmid DNA. ssDNA, CoT DNA, GAPDH DNA and Ub-C DNA at 100 ng input were used as a negative control (see the bottom 2 rows) and, as expected, they show no probe binding. The probe used in FIG. 1 is labeled with DNP phosphoramidite of the formula (II) incorporated into the probe.
FIG. 2 is a photomicrograph showing in situ hybridization results of DNP labeled EGFR RNA hybridization probe embodiment of the invention on a prostate tissue sample at various regions.
FIG. 3 is a photomicrograph showing in situ hybridization results of DNP labeled Ki67-AS RNA hybridization probe embodiment of the invention on a commercially confirmed Ki67 positive tissue at various regions.
FIG. 4 is a photomicrograph showing in situ hybridization results of DNP labeled PTEN-AS RNA hybridization probe embodiment of the invention on a prostate tissue sample using manual (A) and Ventana Discovery Ultra automation staining (B).
FIG. 5 is a photomicrograph showing in situ hybridization results of DNP labeled HER2-AS RNA hybridization probe embodiment of the invention on a prostate tissue sample using manual (A) and Ventana Discovery Ultra automation staining (B).

### DETAILED DESCRIPTION OF THE INVENTION

As used herein, alkyl represents a straight-chain or branched alkyl. By way of example (C₁-C₂₀) alkyl can have 1 to 20 carbon atoms. In an aspect, an alkyl can include, but is not limited to, methyl, ethyl, n-propyl, isopropyl, n-butyl, iso-butyl, sec-butyl, tert-butyl, 1-ethylpropyl, n-pentyl, iso-pentyl, n-hexyl, n-heptyl, n-octyl, n-nonyl, n-decyl, n-undecyl, or n-dodecyl.

As used herein, alkoxy refers to an alkyl group which is singularly bonded to oxygen. As used herein, cycloalkyl refers to a saturated monocyclic carbocyclic group having 3 to 6 carbon atoms and includes for example cyclopropyl, cyclobutyl, cyclopentyl and cyclohexyl.

As used herein, polyether represents a chain having a repeating unit of 1 to 4 carbon atoms and an oxygen. By way of example, the polyether units can include two carbons - (CH₂CH₂O)-, three carbons (CH₂CH₂CH₂O), or four carbons (CH₂CH₂CH₂CH₂O) and the number of repeating polyether units in the chain is 1, 2, 3, 4, 5, 6, 7, 8, or 9.

As used herein, aryl or aromatic group refers to an optionally substituted monocyclic or fused bicyclic or polycyclic ring system having the well-known characteristics of aromaticity, wherein at least one ring contains a completely conjugated pi-electron system. Typically, aryl groups contain 6 to 20 carbon atoms ("C₆-C₂₀ aryl") as ring members, preferably 6 to 14 carbon atoms ("C₆-C₁₄ aryl") or more preferably, 6 to 12 carbon atoms ("C₆-C₁₂ aryl"). By way of example, aromatic groups can include phenyl, pyrrole, furan, anthracene, and pyrene. As used herein, heterocycle refers to a non-aromatic, saturated or partially unsaturated ring system containing the specified number of ring atoms, including at least one heteroatom selected from N, O and S as a ring member, wherein the heterocyclic ring is connected to the base molecule via a ring atom, which may be C or N. Examples of saturated heterocycles are azetidinyl, pyrrolidinyl and piperidinyl.

As used herein, the terms "oligo," "oligonucleotide," "polynucleotide," and "nucleic acid," include polymer sequences of two or more monomers, wherein the monomer is selected from the group consisting of nucleotides (whether DNA or RNA) and nucleotide analogs, wherein the nucleotide analog may be any nucleotide analog known in the art or may be a phosphoramidite compound of the present disclosure, preferably the compound of formula (I) or formula (II). The nucleotides of the present disclosure may be isolated from natural sources, recombinantly produced, or artificially synthesized.

As used herein, a probe refers to a nucleic acid capable of binding to a target nucleic acid of substantially complementary sequence through complementary base pairing, usually through hydrogen bond formation. As used herein, a probe may include natural (i.e. A, G, U, C, or T) or modified bases (7-deazaguanosine, inosine, etc.).

The term "target nucleic acid" or "target sequence" refers to a nucleic acid or nucleic acid sequence which is to be analyzed or detected in a sample. A target can be a nucleic acid to which a probe will hybridize. The probe may or may not be specifically designed to hybridize to the target. It is either the presence or absence of the target nucleic acid that is to be detected, or the amount of the target nucleic acid that is to be quantified. The term target nucleic acid may refer to the specific subsequence of a larger nucleic acid to which the probe is directed or to the overall sequence (e.g., gene or mRNA) it is desired to detect. The difference in usage will be apparent from context.

The term "hybridization" refers to the process in which two single-stranded polynucleotides bind non-covalently to form a stable double-stranded polynucleotide. The resulting double-stranded polynucleotide is a "hybrid." The proportion of the population of polynucleotides that forms stable hybrids is referred to herein as the "degree of hybridization." Hybrids can contain two DNA strands, two RNA strands, or one DNA and one RNA strand.

### Phosphoramidite Compounds of the Present Disclosure

One aspect of the current disclosure is a compound of the formula (I): 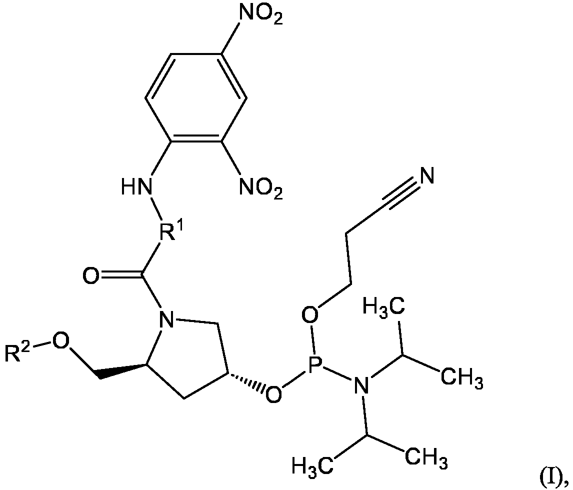
wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and - ((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C1-C6-alkyl, C₃-C₇ cycloalkyl, Ci-C₄dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups;
and wherein R² is C₁-C₂₀-alkyl, preferably C₁-C₆-alkyl, or any suitable protecting group for an alcohol. Preferably R² is 4,4'-dimethoxytrityl (DMT) having the following structure:

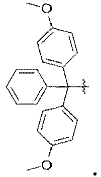

In some embodiments, R¹ is a C₁-C₂₀-alkyl. In another embodiment, R¹ is a substituted or unsubstituted C₁-C₆-alkyl. In a further embodiment, R¹ is an unsubstituted C₅-alkyl. In one embodiment, the compound is (3R,5R)-5-((bis(4-methoxyphenyl)(phenyl)methoxy)methyl)-1-(6-((2,4-dinitrophenyl)amino)hexanoyl)pyrrolidin-3-yl (2-cyanoethyl) diisopropylphosphoramidite of the formula (II):

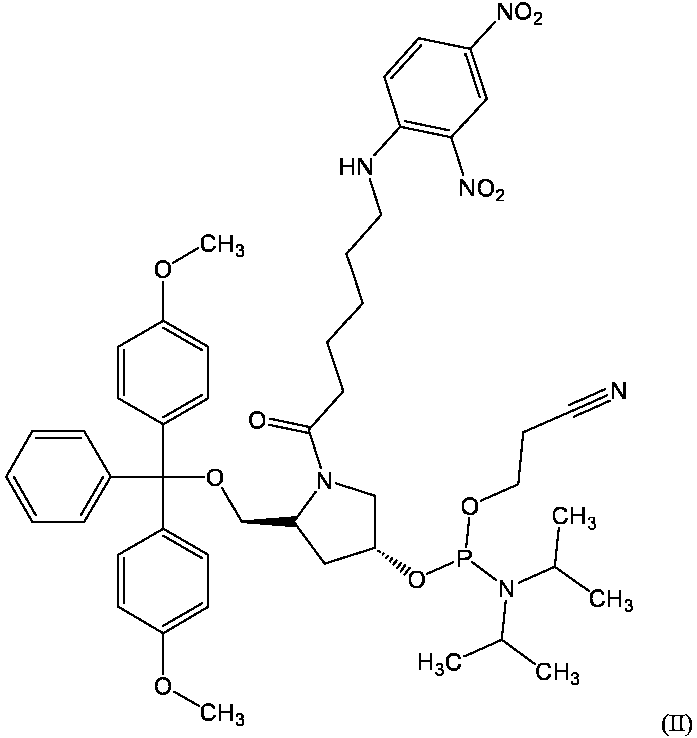

The phosphoramidite compounds of the present disclosure may be used in the synthesis of oligo- and poly-nucleotides, such as oligonucleotide probes. These nucleic acids may be prepared according to standard phosphoramidite synthesis procedure, e.g., automated synthesis. In one embodiment, the present disclosure is directed to an oligo- or poly-nucleotide prepared by reacting the compound of formula (I) with one or more nucleic acids, nucleic acid analogs, or other compounds of formula (I). In one embodiment, the present disclosure concerns a compound of formula (III): 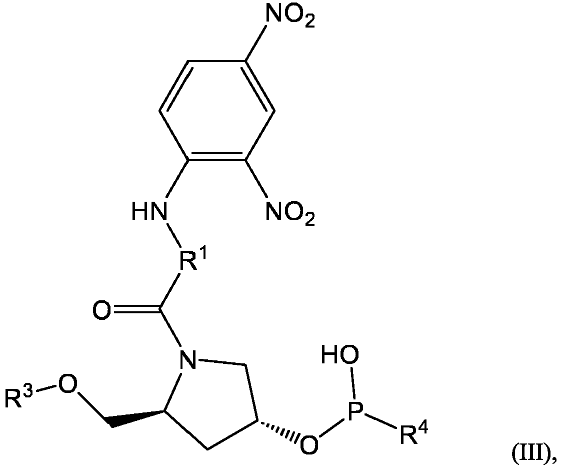
wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and - ((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups;
and wherein R³ and R⁴ are the same or different and R³ is selected from the group consisting of C₁-C₂₀-alkyl, preferably C₁-C₆-alkyl, or any suitable protecting group for an alcohol, preferably 4,4'dimethoxytrityl, a naturally occurring nucleotide, nucleotide analogs and phosphoramidites, and R⁴ is selected from the group consisting of a naturally occurring nucleotide, nucleotide analogs and phosphoramidites, wherein R³ and R⁴ are attached to the compound of formula (III) by phosphodiester bonds. R³ and/or R⁴ may be a monomer or nucleotide that is encompassed within a polynucleotide or oligonucleotide chain. In one embodiment of the present disclosure, the compound of the formula (III) is of the formula (IV):

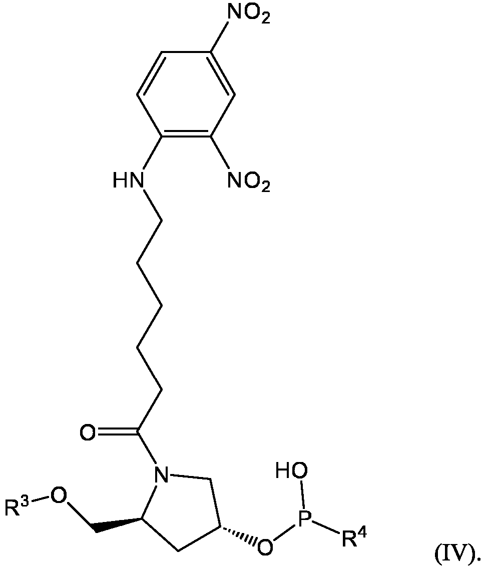

For example, in one embodiment the dinitrophenyl moiety of the present disclosure can be positioned between two nucleotides within the polynucleotide chain such that the polynucleotide has the structure shown here: 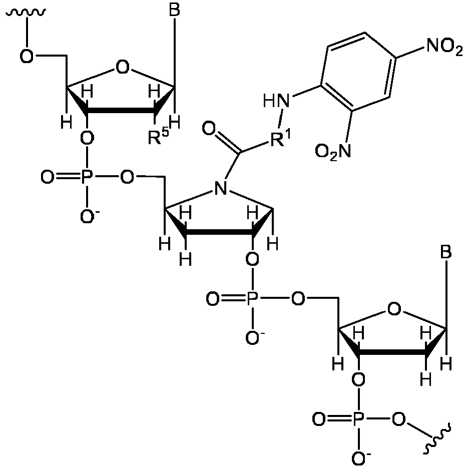
where R⁵ is -OH or hydrogen, where the wavy lines indicate where the sequence is truncated for illustration purposes,
and where B can be 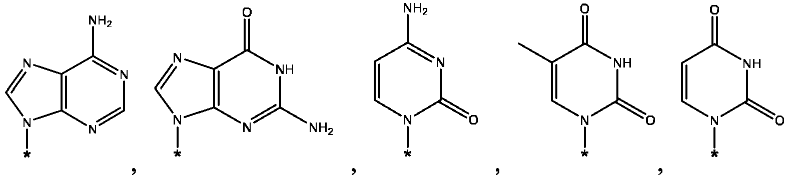 or other substituents as known in the art in which * is the linkage point to the ribose backbone. It should be understood that any combination of natural or unnatural nucleic acids or nucleic acid analogs can be used in the synthetic nucleic acids of the present disclosure. Additionally, and alternatively, the dinitrophenyl moiety can be included at a 3' terminus or a 5' terminus of the synthetic nucleic acids of the present disclosure. Suitable non-naturally occurring nucleic acids and nucleic acid analogs for the compounds and methods of the present disclosure include those having one or more of the following non-natural bases, where -X indicates the linkage to the ribose or deoxyribose backbone:
Iso G: 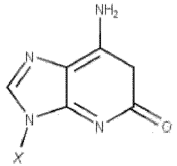
Iso C: 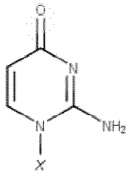
Thymidine Analogs: 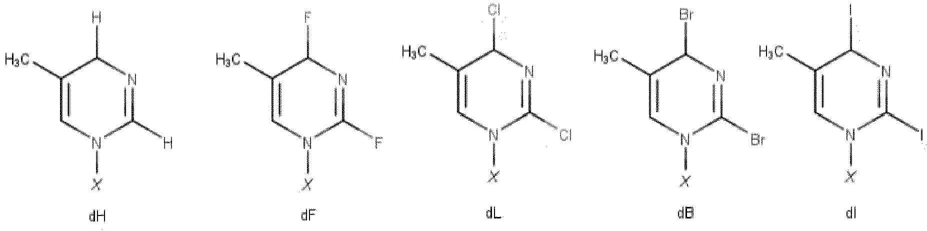
5-Bromo U: 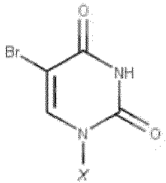
2,6-Diaminopurine: 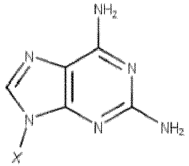
Inosine: 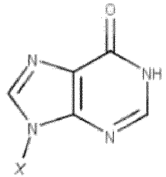
Hydroxymethyl C: 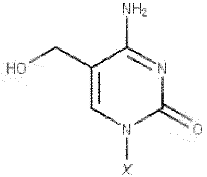
5-Methyl C: 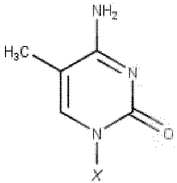
5-Nitro Indole: 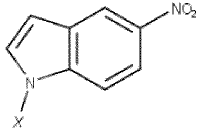
5-Hydroxybutynl-U: 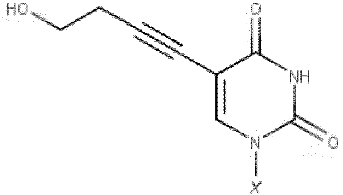
8-Aza-7-deazaguanosine: 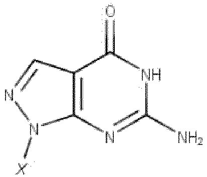
Fluoro Bases, including ¹⁸F- and ¹⁹F-radiolabeled bases:

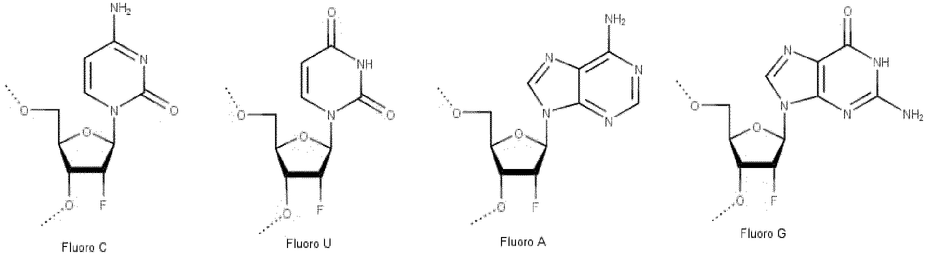

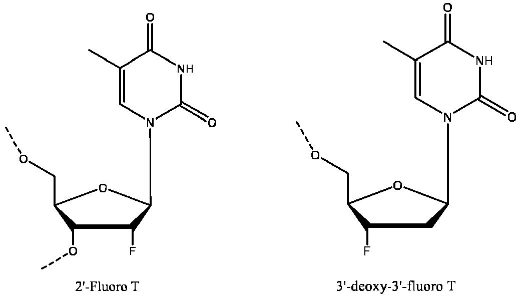

The polynucleotide can include radiolabelled atoms at any position. For example, at least one of ¹⁴C, ¹⁵N, ³H, ¹⁸F, ¹⁹F, ³²P, ³⁵S isotopes can be substituted for non-radiolabeled atoms in the polynucleotide at any position.

### Polynucleotide synthesis with the disclosed phosphoramidite compounds

In an aspect of the current disclosure, methods of synthesizing a polynucleotide, e.g., nucleic acid hybridization probes, comprising a 2,4-dinitrophenyl moiety of the present disclosure are provided. In some embodiments, the methods comprise performing a phosphoramidite nucleic acid sequencing using the disclosed compounds to synthesize a polynucleotide comprising a 2,4-dinitrophenyl moiety.

Automated and manual methods of synthesizing polynucleotides using phosphoramidites are known in the art. The compounds of the present disclosure, which are phosphoramidites comprising nucleoside analogues, may be substituted for standard phosphoramidites in such reactions and incorporated into polynucleotides. The methods may further comprise isolating, enriching, or purifying the polynucleotide to generate an isolated, enriched, or purified polynucleotide, respectively.

In one embodiment, polynucleotide synthesis may comprise (1) linking a first nucleotide to a solid support at the 3' -OH of the nucleotide, and (2) deprotection by detritylation of the 5'-4,4'-dimethoxytrityl (DMT) moiety which is used to link the first nucleotide to, for example, the next nucleotide or nucleoside analog in a polynucleotide. This is followed by (3) coupling: once the DMT has been removed, the free 5'-OH of the solid-support-linked nucleotide or nucleoside is able to react with the next nucleotide or nucleoside, which is added as a phosphoramidite monomer. The diisopropylamino group of the incoming phosphoramidite monomer in the solvent acetonitrile is 'activated' (protonated) by an acidic additive such as tetrazole or ETT [5-(ethylthio)-1H-tetrazole], allowing the 5'-OH to form a bond with the phosphorous of the incoming nucleotide or nucleoside. The mixing is carried out in the fluid lines of the synthesis instrument as the reagents are delivered to the solid support. The activated phosphoramidite is delivered in a many-fold excess over the solid-support-linked nucleoside to drive the reaction to as close to completion as possible. The products include a dinucleoside or dinucleotide with a phosphite triester linkage and a free diisopropylamino group. This is followed by step (4) oxidation: The phosphite triester formed during the coupling reaction is unnatural and unstable; therefore, it must be converted to a more stable phosphorus species prior to the start of the next cycle. Oxidation converts the phosphite triester to the stable phosphate triester. Oxidation of the phosphite triester is achieved with iodine in the presence of water and pyridine. The product is the phosphate triester, which is essentially a standard DNA/RNA backbone with a β-cyanoethyl protecting group on one oxygen of the phosphate group. This is followed by step (5) capping: since 100% coupling efficiency is impossible, there are always some solid-support-linked nucleosides with unreacted 5'-OH. If not blocked, these hydroxyl groups will react during the next cycle, and hence, lead to a missing base. The accumulation of these deletion mutations through successive cycles would create a complex mixture of 'shortmers' that are difficult to purify and therefore could render the oligonucleotide useless in the subsequent application. Capping is required to prevent shortmer accumulation. Acetic anhydride and N-methylimidazole react to form an intermediate in the solvent tetrahydrofuran, which contains a small quantity of pyridine. The mixing is carried out in the fluid lines of the synthesis instrument as the reagents are delivered to the solid support. The product is the solid-support-linked nucleoside with an acetylated 5'-OH (pyridine maintains a basic pH thereby preventing detritylation of the phosphoramidite monomer by the free acetate / acetic acid). This is followed by step (6) deprotection: after cleavage, the solution of oligonucleotide in concentrated aqueous ammonia is heated to remove protecting groups from the bases and phosphates. The oligonucleotide in concentrated aqueous ammonia is heated. The protecting groups can include any appropriate protecting groups for bases, and can for example include: N(6)-benzoyl A, N(4)-benzoyl C, and N(2)-isobutyryl G. The product of the reaction is fully-deprotected A, C, and G bases. The β-cyanoethyl group on the free oxygen of the phosphate must be removed to convert it from a phosphate triester to a phosphate diester (phosphodiester). The cyanoethyl groups are removed in concentrated aqueous ammonia via β-elimination. The reaction is quick because the hydrogen atoms on the carbon adjacent to the electron-withdrawing cyano group are highly acidic. The products are the oligonucleotide with a native phosphodiester backbone and acrylonitrile.

In addition to the standard protecting groups, the labile dimethylformamidyl G and the 'ultramild' protecting groups can be used for modified oligonucleotides that are sensitive to ammonia. The dimethylformamidyl protecting group is typically removed in concentrated aqueous ammonia via heating but in significantly less time than is the isbutyryl group. The ultramild protecting groups include: N(6)-phenoxyacetyl A, N(2)-acetyl C, and N(2)-isopropylphenoxyacetyl G. They are typically removed at room temperature in a concentrated aqueous ammonia / methylamine solution.

Modifications to these methods are known to those of skill in the art and may be used to prepare polynucleic acids from the compounds of the disclosure. The solid support be any one suitable, such as without limitation a polymer support such as polystyrene, a silica support, and a controlled pore glass (CPG).

### Probes and Detection of Target Nucleic Acids Using Polynucleotides Comprising 2,4-Dinitrophenyl Moieties

In an aspect of the current disclosure, the present disclosure is directed to a probe of the formula (III): 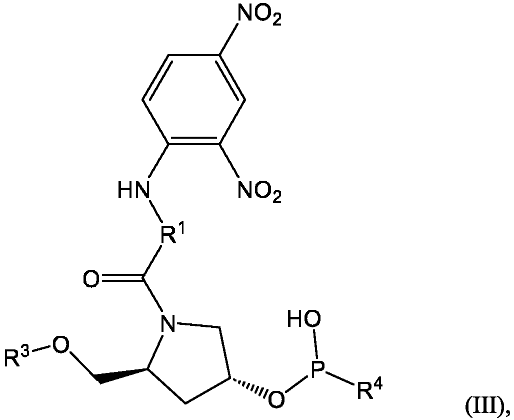 wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and -((CH₂)ₙO)ₘ wherein n is 1, 2, 3, or 4 and m is 1, 2, 3, 4, 5, 6, 7, 8, or 9, wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C1-C6-alkyl, C₃-C₇cycloalkyl, C₁-C₄ dialkylamino, C₆-C₁₄aryl optionally substituted with a halogen and 5-10-membered heteroaryl optionally substituted with a C₆-C₁₀ aryl group and optionally substituted with one to three C₁-C₄ alkyl groups;

and wherein R³ and R⁴ are the same or different and R³ is selected from the group consisting of C₁-C₂₀-alkyl, preferably C₁-C₆-alkyl, or any suitable protecting group for an alcohol, preferably 4,4'dimethoxytrityl, a naturally occurring nucleotide, nucleotide analogs and phosphoramidites, and R⁴ is selected from the group consisting of a naturally occurring nucleotide, nucleotide analogs and phosphoramidites. R³ and/or R⁴ may be a monomer or nucleotide that is encompassed within a polynucleotide or oligonucleotide chain. In one embodiment, R¹ comprises an alkyl group, such as without limitation a saturated or unsaturated C₁-C₂₀-alkyl. In another embodiment, R¹ is C₅-alkyl, C₅-alkenyl, or C₅-alkynyl. In one embodiment, R¹ is unsubstituted or substituted C₅-alkyl or an unsubstituted or substituted aryl. R¹ can be selected for improved solubility, nucleotide binding, or antibody recognition.

In some embodiments, the probe comprises a polynucleotide of formula (IV): 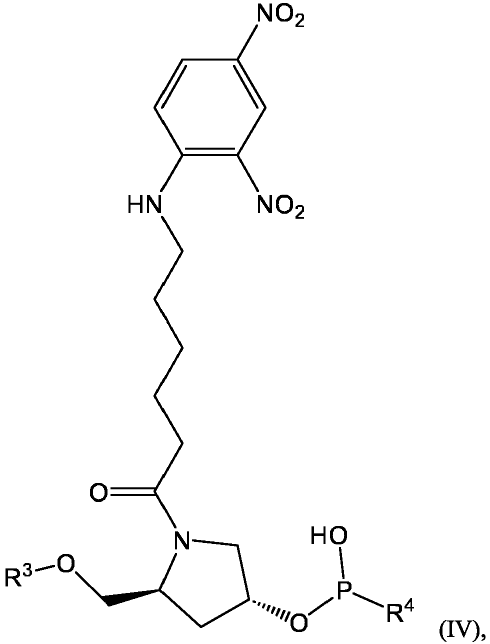 wherein R³ and R⁴ are as defined above in formula (III).

As will be understood by one skilled in the art, the disclosed phosphoramidite compounds may be incorporated into polynucleotides using standard phosphoramidite synthesis methods, resulting in polynucleotides comprising 2,4-dinitrophenyl moieties.

As the 2,4-dinitrophenyl moiety-containing polynucleotides are detectable using anti-2,4-dinitrophenyl detection reagents, e.g., antibodies, the polynucleotides may be used in ISH methods. The detection methods used may be any of those known to one of skill in the art that can be used in conjunction with DNP.

The polynucleotides synthesized with the 2,4-dinitrophenyl moiety according to an aspect as disclosed herein were obtained in higher amount at lower cost that when a commercially available DNP phosphoramidite was used. For example, use of the commercially available P TEG-phosphoramidite from Lumiprobe requires removal of cyanoethyl protection group prior to DMT group deprotection. In contrast, use of the DNP-Phosphoramidite of formula (I) does not require this extra precautionary step as this intra-molecular cyclization reaction in not possible due to proper separation between DMT protection alcohol and cyanoethyl group.

The target nucleic acid sequence may be any natural or non-natural nucleotide sequence. In one embodiment, the probes of the present disclosure are designed to detect a naturally occurring nucleic acid sequence. For the design of such oligo probes of the present disclosure, the nucleic acid sequence of the desired target may be drawn from public sources such as the NCBI database and UCSC genome browser covering most of the transcript variation for the gene of interest. Probe sequences may then be designed with optimized properties, such as a GC content in the range of 30-70 % and Tm in the range of 35-65 °C with the selected target sequences. Presented as an example, SEQ ID NO:1 is the antisense sequence that selectively binds to HPV6 mRNA copies within the desired targets. This sequence served as the basis for preparing oligonucleotide probes for detecting HPV6 that were used in the examples.

Similarly, SEQ ID NO:2 is the antisense sequence that selectively binds to EGFR gene consensus that served as the basis for preparing oligonucleotide probes shown in the examples for detecting mRNA of EGFR. Similar approach was used to prepare probes for hybridization used in the other examples herein.

The nucleic acids such as the probes of the present disclosure may be tagged with a detectable compound directly or indirectly via the DNP moiety. In one embodiment, the nucleic acid of the present disclosure is incubated with an anti-dinitrophenyl antibody-detectable label conjugate ("antibody conjugate") under conditions such that the antibody conjugate selectively binds to the nucleic acid and thereby a detectable label is indirectly incorporated into the nucleic acid or probe of the present disclosure. The detectable label may be fluorescein, horseradish peroxidase, alkaline phosphatase, or any other label or tag known in the field that provides a component for a detection system to identify which nucleic acids in a mixture are hybridized to the probe. Other labeling systems are known that may be used by conjugating to the anti-DNP antibody to detect the hybridized nucleic acids of the present disclosure, such as labeling systems and reagents from Enzo Life Sciences, Inc., Farmingdale NY. Anti-Dinitrophenyl antibodies are commercially available, such as the mouse monoclonal anti-dinitrophenyl antibody IgE isotype sold by Sigma-Aldrich (accessed 12 Jan 2024); DNP monoclonal antibody (LO-DNP-2 and LO-DNP-30) sold by Invitrogen; and a goat DNP polyclonal antibody from Bethyl Laboratories.

In one embodiment, the present disclosure concerns a non-naturally occurring composition of matter comprising a nucleic acid of the formula (III), preferably of the formula (IV), hybridized to a preselected target sequence. In one embodiment, the composition of matter further comprises an anti-DNP antibody-detectable label conjugate that is selectively bound to the nucleic acid of formula (III) or (IV) hybridized to the target sequence.

In one embodiment, the nucleic acids of the present disclosure are used for detection of nucleic acids with *in situ* hybridization. A major advantage of *in situ* hybridization is that it enables maximum use of tissue that is difficult to obtain (e.g., embryos and clinical biopsies). Hundreds of different hybridizations can be performed on the same tissue. Libraries of tissues can be formed and stored in the freezer for future use.

A further embodiment of the invention provides an in vitro hybrid composition of matter that includes:
(i) any of the non-naturally occurring nucleic acid (or probe embodiments) described herein; and
(ii) a naturally occurring (biological) nucleic acid target molecule including the nucleic acid target sequence,
wherein (i) has nucleic acid sequences that are substantially complementary to nucleic acid sequences of the naturally occurring (biological) nucleic acid target molecule and wherein (i) is hybridized to (ii).

A still further embodiment of the invention provides a method for detecting a target nucleic acid sequence in a sample that includes the steps of
providing a non-naturally occurring nucleic acid (or any of the probe embodiments) as described herein;
contacting the non-naturally occurring nucleic acid (or probe embodiment) with an isolated biological sample that may contain nucleic acid molecules that include the preselected nucleic acid target sequence under conditions permitting hybridization of the non-naturally occurring nucleic acid molecule (or probe embodiment) to the nucleic acid molecules including the preselected nucleic acid target sequence if present in the sample; and
detecting label of any of the non-naturally occurring nucleic acid (or probe embodiment) that may be hybridized to the nucleic acid molecules that include the preselected nucleic acid target sequence if present in the sample.

The method may further include a washing step after the contacting step and before the detecting step to remove non-naturally occurring linear nucleic acid molecule/probe that is not specifically hybridized to a target nucleic acid molecule in the sample/specimen. The detecting step may include a 2D or 3D visualization/image capture using microscopy as known in the art.

Methods for conducting polynucleotide hybridization assays have been well developed in the art. Hybridization assay procedures and conditions will vary depending on the application and are selected in accordance with the general binding methods known including those referred to in: Molecular Cloning, A Laboratory Manual, Second Ed., J. Sambrook, et al., Eds., Cold Spring Harbor Laboratory Press, 2012 ("Sambrook et al."); Berger and Kimmel, "Methods in Enzymology," Vol. 152, "Guide to Molecular Cloning Techniques", Academic Press, Inc., San Diego, Calif., 2010; Young and Davis, Proc. Natl. Acad. Sci., U.S.A., 80:1194 (1983), each of which are incorporated herein by reference.

It is appreciated that the ability of two single stranded polynucleotides to hybridize will depend upon factors such as their degree of complementarity as well as the stringency of the hybridization reaction conditions.

As used herein, "stringency" refers to the conditions of a hybridization reaction that influence the degree to which polynucleotides hybridize. Stringent conditions can be selected that allow polynucleotide duplexes to be distinguished based on their degree of mismatch. High stringency is correlated with a lower probability for the formation of a duplex containing mismatched bases. Thus, the higher the stringency, the greater the probability that two single-stranded polynucleotides, capable of forming a mismatched duplex, will remain single-stranded. Conversely, at lower stringency, the probability of formation of a mismatched duplex is increased.

### Miscellaneous

Unless otherwise specified or indicated by context, the terms "a", "an", and "the" mean "one or more." For example, "a molecule" should be interpreted to mean "one or more molecules."

As used herein, "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean plus or minus ≤10% of the particular term and "substantially" and "significantly" will mean plus or minus >10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion additional components other than the components recited in the claims. The term "consisting essentially of' should be interpreted to be partially closed and allowing the inclusion only of additional components that do not fundamentally alter the nature of the claimed subject matter.

All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

All references, including publications, patent applications, and patents, cited herein are hereby incorporated by reference to the same extent as if each reference were individually and specifically indicated to be incorporated by reference and were set forth in its entirety herein.

Preferred aspects of this invention are described herein, including the best mode known to the inventors for carrying out the invention. Variations of those preferred aspects may become apparent to those of ordinary skill in the art upon reading the foregoing description. The inventors expect a person having ordinary skill in the art to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### EXAMPLES

### Synthesis of DNP-Phosphoramidite derivative

Synthesis of DNP-phosphoramidite derivative for oligonucleotide preparation is described below. Other reagents for oligonucleotide synthesis were purchased from commercial sources.

### EXAMPLE 1: Synthesis of 6-[(2,4-Dinitrophenyl)amino] hexanoic acid (DNP X Acid, Compound 1).

An aqueous solution of potassium carbonate (110.6 g K₂CO₃ in 400 mL water) was added to a mixture of caproic acid (36 g, 200 mmol) and 1-fluoro-2,4-dinitrobenzene (52 g, 400 mmol) in acetone (300 mL). After stirred at room temperature of 1 hour, the reaction mixture was acidified to pH 2 by slow addition of concentrated hydrochloric acid. The precipitate was collected by filtration and then washed with 0.1 N hydrochloric acid (1 L) and cold acetone (1 L). The yellow solid was dried under vacuum to provide 56.4 g (95%) product. The chemical structure of Compound 1 is shown below:

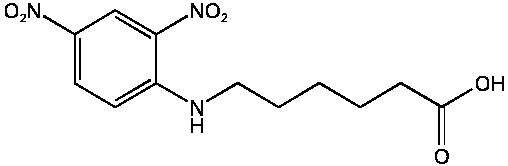

Compound 1 was characterized by LC-MS analysis showing mass observed as 296.03 with calculated value of 296.08.

### EXAMPLE 2: Synthesis of (2S,4R)-4-Hydroxyprolinol Hydrochloride (Compound 2).

Sodium borohydride (25 g, 0.66 mol) was added cautiously into a mixture of (2*S*, 4R)-4-hydroxyproline methyl ester hydrochloride (24 g, 0.13 mol) in dioxane (260 mL) under vigorous stirring. The mixture was heated to reflux, followed by dropwise addition of methanol (90 mL). After the addition, the mixture was refluxed for 4 hours, then stirred at room temperature overnight. The reaction was quenched by adding concentrated hydrochloric acid (100 mL). The solid was removed by filtration, and the filtrate was evaporated to dryness under vacuum. Isopropanol (50 mL) was added to the residue and stirred for 10 min. The insoluble solid was filtered off. After methanol (75 mL) and concentrated HCl (10 mL) were added to the filtrate, the whole mixture was refluxed for 30 min and then evaporated to dryness under vacuum. The residue was recrystallized with isopropanol (300 mL) to provide product (8.5 g) as a white powder. The chemical structure of Compound 2 is shown below:

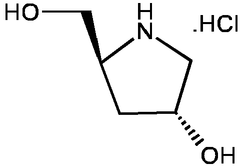

Compound 2 was characterized by LC-MS analysis showing mass observed as 116.07 with calculated value of 116.07.

### EXAMPLE 3: Synthesis of Compound 3

TSTU (11.6 g, 38.5 mmol) and DIPEA (13.5 g, 105 mmol) were added sequentially into a mixture of 6-[(2,4-Dinitrophenyl)amino]hexanoic acid (10.4 g, 35 mmol) in acetonitrile (180 mL). The reaction was stirred at room temperature and monitored by TLC (dichloromethane/methanol, 95/5, v/v). The reaction was done in about 30 min. (2*S*,4*R*)-4-Hydroxyprolinol Hydrochloride (5.4 g, 35 mmol) was added into the above reaction mixture. The mixture was stirred at room temperature and monitored by TLC (dichloromethane/methanol, 95/5, v/v). After stirred at room temperature for 3 hours, the mixture was evaporated under vacuum. The residue was dissolved in dichloromethane (100 mL), and then added slowly with stirring into ethyl ether (800 mL). The ethyl ether layer was discarded. The precipitate sticky oil was again dissolved in dichloromethane (100 mL) and then added to ethyl ether (800 mL). The precipitate oil was collected and purified by flash chromatography (300 g silica gel column; 0% to 10% methanol in dichloromethane in 20 column volumes). The fractions were checked by TLC (10% methanol in dichloromethane), combined and evaporated to give product (18.4 g). The chemical structure of Compound 3 is shown below:

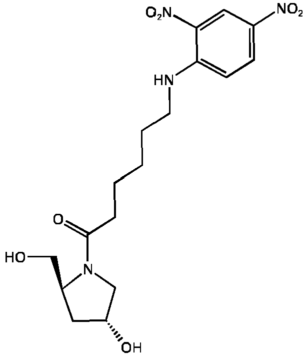

Compound 3 was characterized by LC-MS analysis showing mass observed as 395.16 with calculated value of 395.15.

### EXAMPLE 4: Synthesis of Compound 4

DMAP (catalytic amount) was added to a solution of starting diol (18.4 g, 46.5 mmol) in pyridine (150 mL). A solution of DMTrCl (17.3 g, 51.0 mmol) in pyridine (100 mL) was added dropwise while the reaction mixture was cooled by water bath. The mixture was stirred at room temperature overnight. The reaction was monitored by TLC (5% methanol in dichloromethane). The solvent was removed under vacuum. The residue was dissolved in dichloromethane (300 mL) and washed with brine (2 × 200 mL), dried with anhydrous sodium sulfate. The solvent was removed under vacuum. The residue was purified by flash chromatography (440 g silica gel, 0% to 10% of methanol in dichloromethane containing 5% of triethylamine). The fractions were checked by TLC (5% methanol in dichloromethane), combined and evaporated under vacuum to give product as yellow solid (15 g). The chemical structure of Compound 4 is shown below:

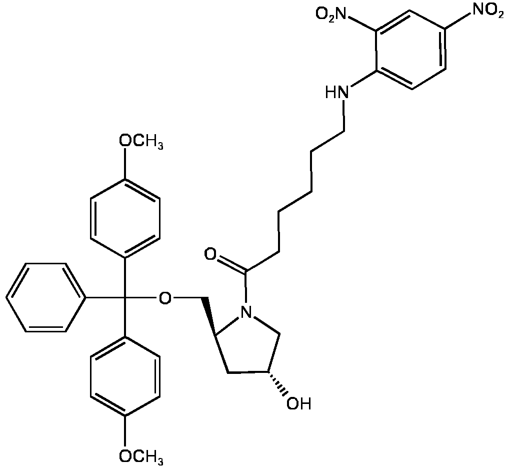

Compound 4 was characterized by LC-MS analysis showing mass observed as 697.35 with calculated value of 697.29.

### EXAMPLE 5: Synthesis of Compound of Formula (II)

Tetrazole (0.45 M in acetonitrile, 3.6 mL, 1.6 mmol) was added to a solution of starting alcohol (4.0 g, 5.7 mmol) and 2-cyanoethyl *N,N,N',N'*-tetraisopropyl-phosphorodiamidite (2.1 g, 6.9 mmol) in dichloromethane (60 mL) under argon. The mixture was stirred at room temperature overnight. TLC (5% methanol in dichloromethane) showed the reaction was complete. The mixture was washed with saturated sodium bicarbonate solution (2 × 100 mL) and brine (100 mL), then dried with anhydrous sodium sulfate. The solvent was removed under vacuum. The residue was purified by flash chromatography (120 g silica gel column, 2% triethylamine in toluene as mobile phase). The fractions were checked by TLC (5% methanol in dichloromethane), combined and then evaporated under vacuum. The yellow solid (6.7 g) obtained was dried under high vacuum for 1 day and then stored under argon at -20 °C. HPLC showed purity over 99% of the combined isomers. The chemical structure of the compound of formula (II) is shown below:

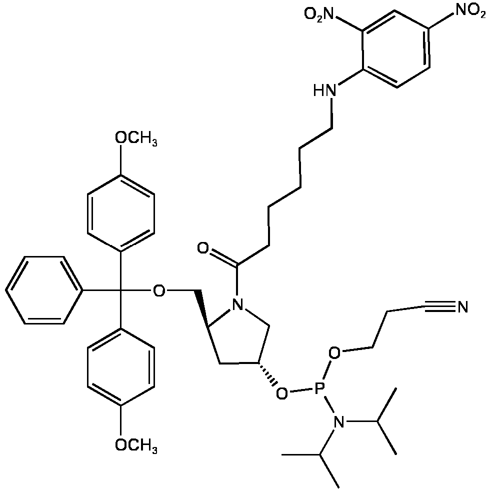

The compound of formula (II) produced was characterized by LC-MS analysis showing mass observed as 897.36 with calculated value of 897.39. The purity of the compound of formula (II) was determined by HPLC (reversed-phase) and was found to be 99.62%.

### EXAMPLE 6: Modified Oligonucleotide Synthesis Containing the Compound of Formula (II)

A plurality of oligonucleotides was prepared using the Dr. Oligo 48 synthesizer, employing universal CPG support at a synthesis scale of 200 nmol. The synthesis process adhered to a protocol supplied by the manufacturer of the aforementioned apparatus. To incorporate DNP moieties into the oligonucleotides for the purpose of establishing an antibody recognition locus, Compound of formula (II) was introduced into the synthesis, alongside other adjusted phosphoramidites of conventional bases. Subsequent to the synthetic procedure, detachment of the oligonucleotides from the solid surface and deprotection of the nucleotide bases were accomplished utilizing an aqueous ammonia medium. The resulting crude oligonucleotides were subsequently purified on reverse phase silica columns using standard methods. Evaluation and characterization of these oligonucleotides were conducted through Electrospray Ionization Mass Spectrometry (ESI-MS). A composite assortment of 12 to 24 (or higher) highly specific, labeled oligonucleotides, in a buffer solution of TE-8, formed the Hybridization Probe Stock Solution. These oligonucleotides were designed to target distinct nucleic acid sequences such as DNA and various forms of RNA. An example of such oligonucleotide probes directing towards EGFR mRNA target is shown below in the list of oligo probe sequences (m is 2'-methoxy modification, r is ribo bases).

### EXAMPLE 7: Modified Oligonucleotide Synthesis Containing Biotin Moiety

Procedure shown in Example 6 was followed to synthesize oligonucleotides containing Biotin modification using commercially available phosphoramidite derivatives.

### EXAMPLE 8: Modified Oligonucleotide Synthesis Containing Digoxigenin Moiety

Due to incompatibility of Digoxigenin during solid phase oligonucleotide synthesis an alternate method involving copper-catalyzed azide-alkyne cycloaddition reaction (CuAAC) was performed. Desired oligonucleotides sequences were synthesized using commercially available alkyne phosphoramidite that were then conjugated to Digoxigenin-azide (Enzo Life Sciences) using CuAAC. The resulting crude oligonucleotides were subsequently purified on reverse phase silica columns using standard methods. Evaluation and characterization of these oligonucleotides were conducted through Electrospray Ionization Mass Spectrometry (ESI-MS).

### EXAMPLE 9: Dot-Blot Staining for the Binding of Oligonucleotide Probes with Plasmid Targets

For the purpose of assessing binding between oligonucleotide probes and plasmid targets, a succession of plasmid target dilutions was applied onto a MaxSense^{®} hybridization membrane (Enzo Catalog Number 45701) subsequent to the implementation of standard membrane blocking procedures. Following this, the membrane was subjected to treatment with HPV6 oligonucleotide RNA probes for a period of 2 hours at a temperature of 50 °C, allowing for hybridization to occur. The HPV6 oligonucleotide RNA probe was labeled with biotin phosphoramidite (hydroxyprolinol) that was purchased from Lumiprobe Corporation, Hunt Valley, Maryland, which had the following structure:

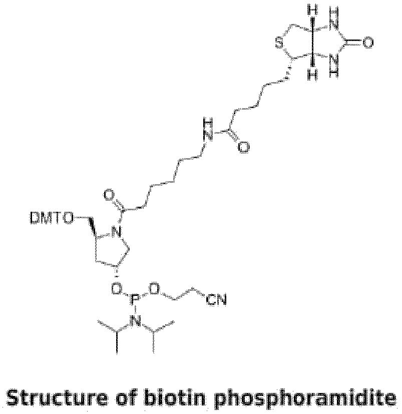

Subsequent to the hybridization step, the membrane was subjected to a series of washes using a TBS-T buffer, followed by incubation with a linker antibody directed against Biotin, maintained for a duration of 1 hour at room temperature. Further washing with TBS-T buffer succeeded by an incubation with Poly View Plus-HRP (anti-rabbit, Enzo). Following additional washing with TBS-T buffer, signal detection was executed utilizing a DAB chromogen (Enzo). Notably, the detected signal exhibited a clear dependence on the concentration of the plasmid target. No binding interaction was observed with the single-stranded DNA (ssDNA) utilized as a negative control.

### EXAMPLE 10: Examples of dinitrophenyl nucleosides and nucleotides used in polynucleotide synthesis of the present disclosure

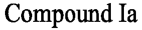

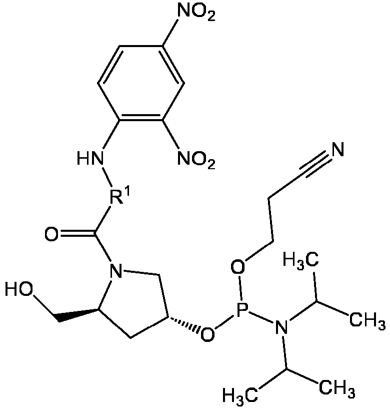

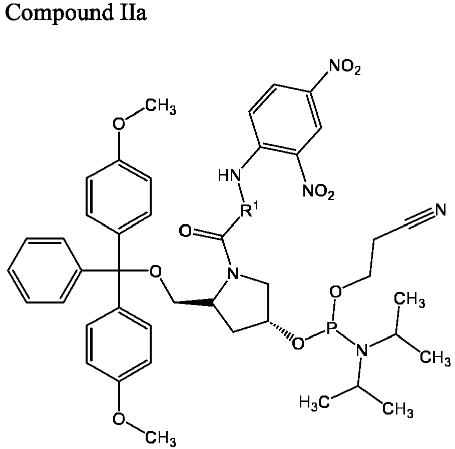

### EXAMPLE 11: ISH (in situ hybridization) of various probes of the invention on tissue slides

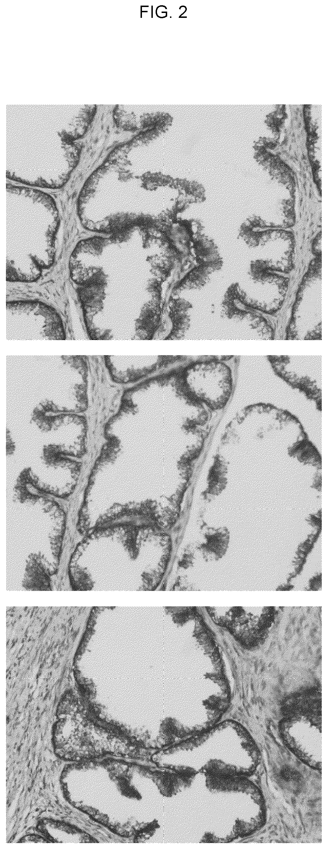

The methodology employed for the detection of signals through In Situ Hybridization (ISH) encompasses a series of procedural stages, including slide preparation and pre-treatment, probe hybridization and its subsequent wash, along with primary and secondary antibody incubation, culminating in either Horseradish Peroxidase (HRP) or Alkaline Phosphatase (AP) detection. The preparation and pre-treatment of tissue slides adhered to conventional procedures well-documented in relevant literature. The hybridization of the probes was executed at a temperature of 45°C for a duration of 2 hours. Subsequent to the hybridization procedure, a sequence of washing steps was performed, followed by the application of linker and detection antibodies. Ultimately, signal generation was obtained utilizing DAB chromogen system. The outcomes of this process are shown and elaborated upon in FIG. 2 through FIG. 6, wherein HPV6, EGFR, and Ki67 positive tissues are depicted. In the data reported in FIG. 2 through FIG. 6, the oligonucleotide RNA probe was labeled with the compound of formula (II) prepared by the process of the examples.

FIG. 2 is a photomicrograph showing in situ hybridization results of DNP labeled EGFR RNA hybridization probe embodiment of the invention on a prostrate tissue at various regions. A clean distinct staining pertaining to EGFR target was obtained with the probes of this invention (see FIG. 2).

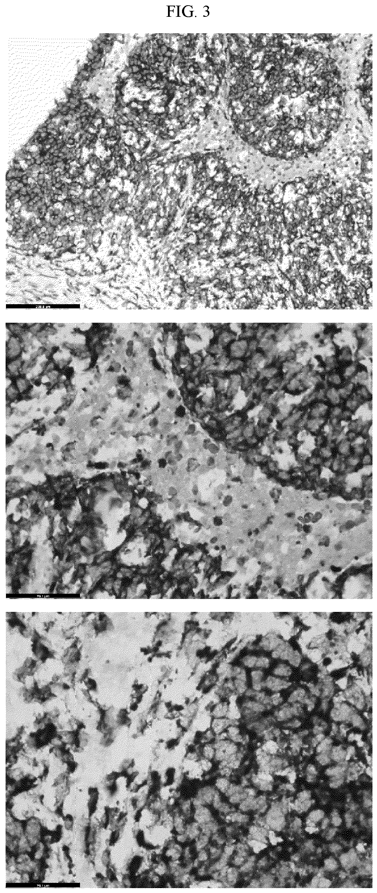

FIG. 3 is a photomicrograph showing in situ hybridization results of DNP labeled Ki67 RNA hybridization probe embodiment of the invention on a commercially confirmed Ki67 positive tissue at various regions. The DNP labeled probe for Ki67 produced distinct staining pattern pertaining to the target.

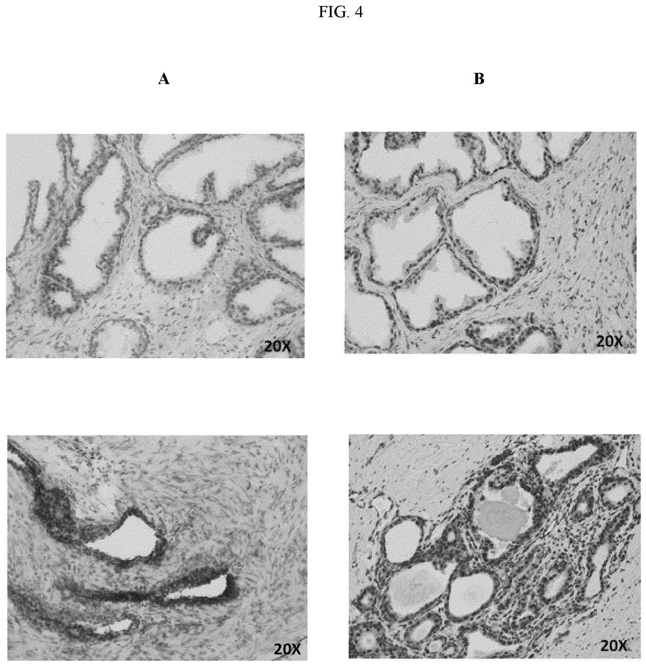

FIG. 4 is a photomicrograph showing in situ hybridization results of DNP labeled PTEN-AS RNA hybridization probe embodiment of the invention on a prostate tissue sample using manual (A) and automation staining (B).

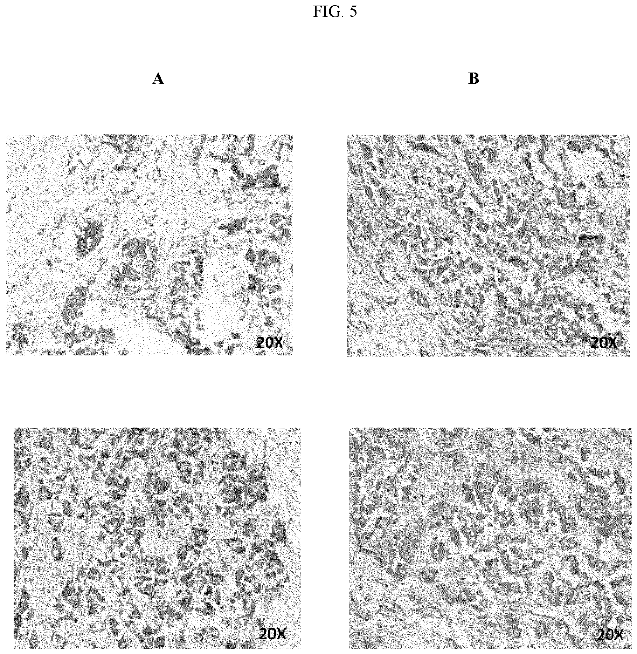

FIG. 5 is a photomicrograph showing in situ hybridization results of DNP labeled HER2-AS RNA hybridization probe embodiment of the invention on a prostate tissue sample using manual (A) and automation staining (B).

The probe sequences used in the data reported in FIGS. 2-5 are these:
**Oligo Probe Sequences:**
**A) EGFR-Antisense**

| **SEQ ID NO:** | **Sequence** |
|---|---|
| **3** | (Linker)mAmGmA mUmCmC mUmCmA mAmGmA mGmAmG (Loop) mCmUmU mGmGmU mUmGmG mGmAmG mCmUmU (Linker) |
| **4** | (Linker)mCmUmC mCmAmC mUmGmG mGmUmG mUmAmA (Loop) mGmAmG mGmCmU mCmCmA mCmAmA mGmCmU (Linker) |
| 5 | (Linker)mUmUmC mCmGmA mAmCmG mAmUmG mUmGmG (Loop) mCmGmC mCmUmU mCmGmC mAmUmG mAmAmG (Linker) |
| 6 | (Linker)mUmUmC mCmGmA mAmCmG mAmUmG mUmGmG (Loop) mCmGmC mCmUmU mCmGmC mAmUmG mAmAmG (Linker) |
| **7** | (Linker) mCmCmA mGmUmG mCmAmU mCmCmG mUmAmG (Loop) mGmUmG mCmAmG mUmUmU mGmGmA mUmGmG (Linker) |
| **8** | (Linker) mGmUmC mUmGmC mGmUmA mCmUmU mCmCmA (Loop) mGmAmC mCmAmG mGmGmU mGmUmU mGmUmU (Linker) |
| **9** | (Linker) mAmUmA mCmAmG mUmUmG mUmCmU mGmGmU (Loop) mCmCmC mCmGmU mCmCmU mGmUmG mCmAmG (Linker) |
| **10** | (Linker) mGmCmA mCmUmC mUmGmG mGmUmG mGmCmA (Loop) mCmUmG mUmAmU mGmCmA mCmUmC mAmGmA (Linker) |
| **11** | (Linker) mUmUmG mGmCmU mCmAmC mCmCmU mCmCmA (Loop) mGmAmA mGmGmU mUmGmC mAmCmU mUmGmU (Linker) |
| **12** | (Linker) mAmUmU mUmUmG mGmUmU mUmUmC mUmGmA (Loop) mCmCmG mGmAmG mGmUmC mCmCmA mAmAmC (Linker) |
| **13** | (Linker) mUmUmA mUmCmU mCmCmU mUmGmA mGmGmG (Loop) mAmGmC mGmUmA mAmUmC mCmCmA mAmGmG (Linker) |
| **14** | (Linker) mGmGmC mUmGmA mCmGmA mCmUmG mCmAmA (Loop) mGmAmG mAmAmA mAmCmU mGmAmC mCmAmU (Linker) |
| **15** | (Linker) mGmUmC mCmUmG mCmCmG mCmGmU mAmUmG (Loop) mAmUmU mUmCmU mAmGmG mUmUmC mUmCmA (Linker) |
| **16** | (Linker) mGmGmC mCmAmA mGmCmC mUmGmA mAmUmC (Loop) mAmGmC mAmAmA mAmAmC mCmCmU mGmUmG (Linker) |
| **17** | (Linker) mCmAmG mAmAmU mAmUmC mCmAmG mUmUmC (Loop) mCmUmG mUmGmG mAmUmC mCmAmG mAmGmG (Linker) |
| **18** | (Linker) mCmUmA mAmAmU mGmCmC mAmCmC mGmGmC (Loop) mAmGmG mAmUmG mUmGmG mAmGmA mUmCmG (Linker) |
| **19** | (Linker) mCmUmA mUmUmC mCmGmU mUmAmC mAmCmA (Loop) mCmUmU mUmGmC mGmGmC mAmAmG mGmCmC (Linker) |
| **20** | (Linker) mUmCmG mCmAmC mUmUmC mUmUmA mCmAmC (Loop) mUmUmG mCmGmG mAmCmG mCmCmG mUmCmU (Linker) |

**B) Ki-67-Antisense**

| **SEQ ID NO:** | **Sequence** |
|---|---|
| **21** | (Linker) mGmGmA mGmGmG mCmUmU mGmCmA mGmAmG (Loop) mCmAmU mUmUmA mUmCmA mGmAmU mGmGmC (Linker) |
| **22** | (Linker) mCmAmC mAmUmU mUmCmC mUmCmC mAmAmA (Loop) mAmCmU mCmUmC mUmGmA mGmGmU mGmGmG (Linker) |
| **23** | (Linker) mCmAmU mGmUmG mCmUmU mGmUmC mAmAmC (Loop) mUmGmC mGmGmU mUmGmC mUmCmC mUmUmC (Linker) |
| **24** | (Linker) mAmGmU mUmGmU mUmGmA mGmCmA mCmUmC (Loop) mUmGmU mAmGmG mGmUmC mGmAmG mCmAmG (Linker) |
| **25** | (Linker)mAmUmU mGmAmC mUmGmU mGmAmA mCmUmU (Loop) mCmGmC mCmCmA mCmAmG mGmCmU mUmCmU (Linker) |
| **26** | (Linker) mCmUmC mUmUmU mGmAmA mGmGmC mAmGmG (Loop) mUmUmG mCmCmA mCmUmC mUmUmU mCmUmC (Linker) |
| **27** | (Linker) mGmGmC mGmUmU mGmAmU mCmAmC mUmGmG (Loop) mCmAmA mCmUmG mGmAmG mUmUmU mUmCmC (Loop) |
| **28** | (Linker) mCmAmG mGmUmG mGmAmG mUmGmU mGmCmA (Loop) mUmUmA mCmCmA mGmAmG mAmCmU mUmUmC (Linker) |
| **29** | (Linker) mCmCmC mAmCmC mAmAmA mGmGmA mCmAmC (Loop) mAmCmG mCmCmU mUmCmU mUmUmU mCmAmA (Linker) |
| **30** | (Linker) mAmGmA mAmGmC mUmAmG mAmUmC mUmUmG (Loop) mAmGmA mCmAmC mGmAmC mGmUmG mCmUmG (Linker) |
| **31** | (Linker) mCmAmA mUmAmC mCmCmC mUmUmC mCmAmA (Loop) mAmCmA mAmGmC mAmGmG mUmGmC mUmGmA (Linker) |
| **32** | (Linker) mUmCmG mAmCmC mCmCmG mCmUmC mCmUmU (Loop) mUmUmG mAmUmA mGmUmA mAmCmC mAmGmG (Linker |

**C) PTEN-Antisense**

| **SEQ ID NO:** | **Sequence** |
|---|---|
| **33** | (Linker) mCmCmU mCmAmC mAmGmC mGmGmC mUmCmA (Loop) mAmCmU mCmUmC mAmAmA mCmUmU mCmCmA (Linker) |
| **34** | (Linker) mCmGmA mUmCmU mCmUmU mUmGmA mUmGmA (Loop) mUmGmG mCmUmG mUmCmA mUmGmU mCmUmG (Linker) |
| **35** | (Linker) mCmUmA mGmCmU mGmUmG mGmUmG mGmGmU (Loop) mUmAmU mGmGmU mCmUmU mCmAmA mAmAmG (Linker) |
| **36** | (Linker) mCmCmC mCmAmU mAmGmA mAmAmU mCmUmA (Loop) mGmGmG mCmCmU mCmUmU mGmUmG mCmCmU (Linker) |
| **37** | (Linker) mAmCmU mUmGmU mCmUmU mCmCmC mGmUmC (Linker) mGmUmG mUmGmG mGmUmC mCmUmG mAmAmU (Linker) |
| **38** | (Linker) mUmAmU mCmAmC mCmAmC mAmCmA mCmAmG (Loop) mGmUmA mAmCmG mGmCmU mGmAmG mGmGmA (Linker) |
| **39** | (Linker) mUmUmU mCmUmG mAmGmG mUmUmU mCmCmU (Loop) mCmUmG mGmUmC mCmUmG mGmUmA mUmGmA (Linker) |
| **40** | (Linker) mCmCmU mCmUmG mGmAmU mUmUmG mAmCmG (Loop) mGmCmU mCmCmU mCmUmA mCmUmG mUmUmU (Linker) |
| **41** | (Linker) mCmAmU mUmCmU mCmUmG mGmAmU mCmAmG (Loop) mAmGmU mCmAmG mUmGmG mUmGmU mCmAmG (Linker) |
| **42** | (Linker) mGmCmA mUmUmC mCmCmU mCmCmA mUmUmC (Loop) mCmCmC mUmAmA mCmCmC mGmAmA mUmAmC (Linker) |
| **43** | (Linker) mAmCmA mGmCmG mGmCmC mAmAmU mUmCmC (Loop) mUmCmG mAmAmU mGmUmC mCmAmG mAmUmU (Linker) |
| **44** | (Linker) mGmAmA mGmCmU mUmGmC mAmGmG mCmAmG (Loop) mCmAmC mAmUmG mAmAmG mCmAmU mCmCmA (Linker) |
| **45** | (Linker) mUmGmA mAmAmU mUmGmA mAmGmC mCmCmU (Loop) mAmGmU mCmCmC mAmAmC mUmCmG mGmGmG (Linker) |
| **46** | (Linker) mAmGmA mCmCmA mCmAmG mCmUmA mGmUmG (Loop) mAmAmC mAmAmU mUmUmC mAmGmC mAmCmC (Linker) |
| **47** | (Linker) mAmAmA mGmCmC mCmCmC mAmCmU mUmUmA (Loop) mGmUmG mCmAmC mAmGmU mUmCmC mAmCmC (Linker) |
| **48** | (Linker) mCmCmU mCmAmG mUmGmC mAmUmA mGmCmA (Loop) mUmUmU mAmCmA mCmAmC mAmGmA mGmCmC (Linker) |
| **49** | (Linker) mGmCmU mUmAmA mCmAmA mCmCmA mGmAmG (Loop) mUmAmC mUmAmC mCmAmC mCmAmA mAmGmC (Linker) |
| **50** | (Linker) mGmAmC mAmCmA mCmAmC mAmCmA mCmAmC (Linker) mAmCmU mUmAmC mAmAmA mCmUmG mCmAmC (Linker) |

**D) HER2-Antisense**

| **SEQ ID NO:** | **Sequence** |
|---|---|
| **51** | (Linker) mCmAmG mGmUmA mGmGmU mGmAmG mUmUmC (Loop) mCmAmG mGmUmU mUmCmC mCmUmG mCmAmC (Linker) |
| **52** | (Linker) mUmGmG mGmAmC mCmUmG mCmCmU mCmAmC (Loop) mUmUmG mGmUmU mGmUmG mAmGmC mGmAmU (Linker) |
| **53** | (Linker) mCmAmG mUmGmU mGmAmG mAmGmC mCmAmG (Loop) mCmUmG mGmUmU mGmUmU mCmUmU mGmUmG (Linker) |
| **54** | (Linker) mCmAmG mGmCmU mCmUmG mAmCmA mAmUmC (Loop) mCmUmC mAmGmA mAmCmU mCmUmC mUmCmC (Linker) |
| **55** | (Linker) mAmGmG mCmAmG mUmCmA mCmAmC mAmGmC (Loop) mUmGmG mCmGmC mCmGmA mAmUmG mUmAmU (Linker) |
| **56** | (Linker) mUmGmG mAmUmA mUmUmG mGmCmA mCmUmG (Loop) mGmUmA mAmCmU mGmCmC mCmUmC mAmCmC (Linker) |
| **57** | (Linker) mUmCmC mGmGmC mCmAmU mGmCmU mGmAmG (Loop) mAmUmG mUmAmU mAmGmG mUmAmA mCmCmU (Linker) |
| **58** | (Linker) mGmCmA mGmAmA mUmUmC mGmUmC mCmCmC (Loop) mGmGmA mUmUmA mCmUmU mGmCmA mGmGmU (Linker) |
| **59** | (Linker)mAmCmG mAmAmG mCmAmG mAmGmG mUmGmG (Loop) mGmUmG mUmUmA mUmGmG mUmGmG mAmUmG (Linker) |
| **60** | (Linker) mAmAmA mCmAmG mUmGmC mCmUmG mGmCmA (Loop) mUmUmC mAmCmA mUmAmC mUmCmC mCmUmG (Linker) |
| **61** | (Linker) mGmUmC mCmAmA mAmAmC mAmGmG mUmCmA (Loop) mCmUmG mAmGmC mCmAmU mUmCmU mGmGmG (Linker) |
| **62** | (Linker) mCmAmU mCmUmG mGmAmA mAmCmU mUmCmC (Loop) mAmGmA mUmGmG mGmCmA mUmGmU mAmGmG (Linker) |
| **63** | (Linker) mUmGmA mUmGmA mGmGmA mUmCmC mCmAmA (Loop) mAmGmA mCmCmA mCmCmC mCmCmA mAmGmA (Linker) |
| **64** | (Linker) mUmCmU mCmUmU mUmCmA mGmGmA mUmCmC (Loop) mGmCmA mUmCmU mGmCmG mCmCmU mGmGmU (Linker) |
| **65** | (Linker) mAmGmC mUmCmA mUmCmC mCmCmU mUmGmG (Loop) mCmAmA mUmCmU mGmCmA mUmAmC mAmCmC (Linker) |
| **66** | (Linker) mUmGmU mCmAmU mCmGmU mCmCmU mCmCmA (Loop) mGmCmA mGmUmG mAmGmC mGmGmU mAmGmA (Linker) |
| **67** | (Linker) mGmUmA mCmUmG mUmGmG mGmGmU mCmCmU (Loop) mCmAmC mUmGmU mAmCmC mGmCmU mGmUmA (Linker) |
| **68** | (Linker) mGmUmU mCmUmU mAmGmA mCmAmC mUmCmC (Loop) mCmUmU mAmGmG mGmCmC mGmCmU mUmCmC (Linker) |

E) Linker Sequence at 5'-end of the probes:
T(DNP)T T(DNP)T T(DNP)T T
F) Linker Sequence at 3'-end of the probes:
TT(DNP) TT(DNP) TT(DNP) T
G) Loop Sequence in the middle of the probes (from 5'-3' end):
TT(DNP) TT(DNP) TT(DNP) TT
"DNP" refers to the compound of formula (II) incorporated into the sequence.

## Claims

1. A compound represented by the structure of formula (I): 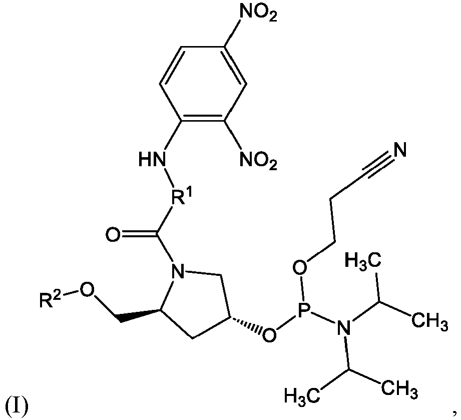
wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and - ((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄ dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups and
wherein R² is C₁-C₂₀-alkyl, preferably C₁-C₆-alkyl, or any suitable protecting group for an alcohol, preferably 4,4'dimethoxytrityl.

2. The compound of claim 1, wherein R¹ is a C₁-C₂₀-alkyl, an unsubstituted C₁-C₆-alkyl, or an unsubstituted C₅-alkyl.

3. The compound of claim 2, wherein the compound is represented by the formula:

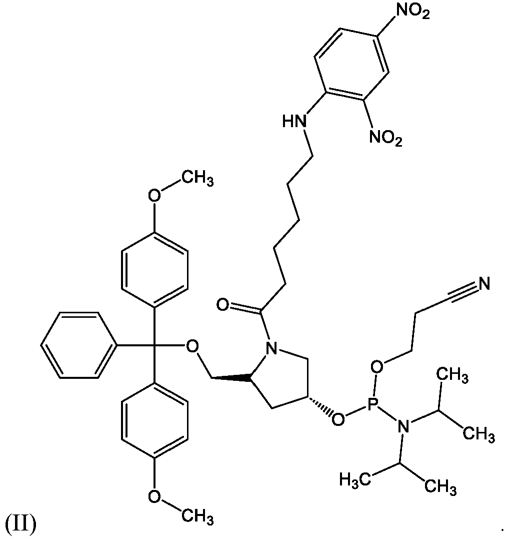

4. A method of preparing a polynucleotide, the method comprising:
a. providing a first oligonucleotide directly or indirectly linked to a solid support at the 3'-OH of the oligonucleotide sugar moiety and having a free 5' -OH group;
b. activating the compound of any one of claims 1-4, providing the activated compound to the first oligonucleotide to form a phosphite triester bond to form a second oligonucleotide;
c. oxidizing the second polynucleotide;
d. deprotecting the second polynucleotide to remove R²;
e. optionally activating a nucleotide, non-naturally occurring nucleotide or phosphoramidite and coupling to the second polynucleotide, oxidizing the resulting third polynucleotide, and deprotecting the third polynucleotide; and
f. cleaving the resulting polynucleotide from the solid support.

5. The method of claim 4, wherein the phosphoramidite monomer is a ribonucleotide or analog thereof.

6. The method of any one of claims 4 or 5, further comprising isolating, enriching, or purifying the polynucleotide to generate an isolated, enriched, or purified polynucleotide, respectively.

7. A polynucleotide made by the method of any one of claims 4 or 5.

8. A polynucleotide, optionally, a nucleic acid hybridization probe, comprising the compound represented by the formula: 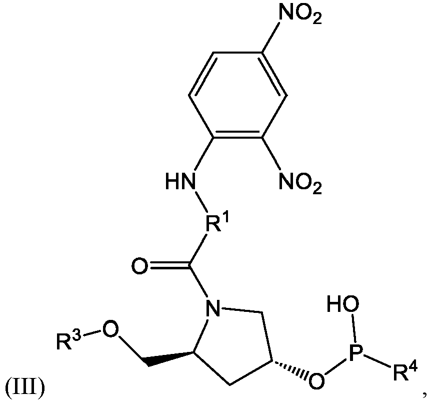
wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and - ((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄ dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups and
wherein R³ and R⁴ are the same or different and R³ is selected from the group consisting of C₁-C₂₀-alkyl, preferably C₁-C₆-alkyl, or any suitable protecting group for an alcohol, preferably 4,4'dimethoxytrityl, a naturally occurring nucleotide, nucleotide analogs and phosphoramidites, and R⁴ is selected from the group consisting of a naturally occurring nucleotide, nucleotide analogs and phosphoramidites
wherein R³ and R⁴ are attached to the compound of formula (III) by phosphodiester bonds.

9. The polynucleotide of claim 8, wherein R¹ comprises a saturated or unsaturated C₁-C₂₀-alkyl, R¹ is an unsubstituted C₁-C₆-alkyl, or wherein R¹ is an unsubstituted or substituted C₅-alkyl or an unsubstituted or substituted aryl.

10. The polynucleotide of claim 9, wherein the polynucleotide comprises a compound of formula (IV):

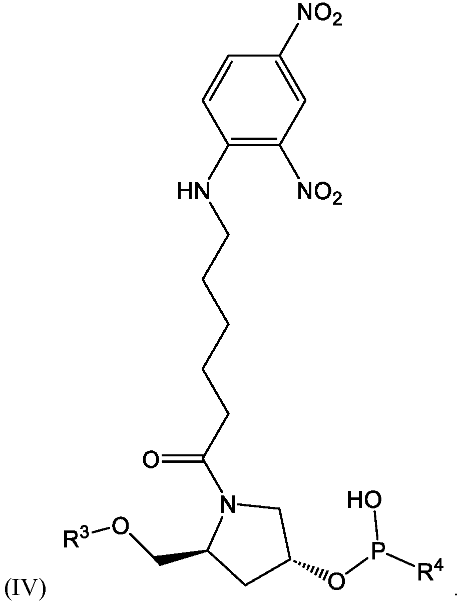

11. A polynucleotide comprising a compound represented by the formula: 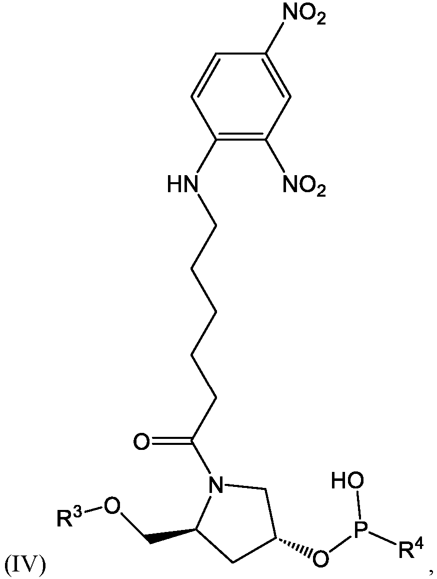
wherein R³ and R⁴ are the same or different and are a naturally occurring nucleotide, or are monomers selected from the group consisting of a second compound of formula (IV), nucleotide analogs and phosphoramidites, and
wherein R³ and R⁴ are attached to the compound of formula (III) by phosphodiester bonds.

12. A method of making a compound represented by the formula: 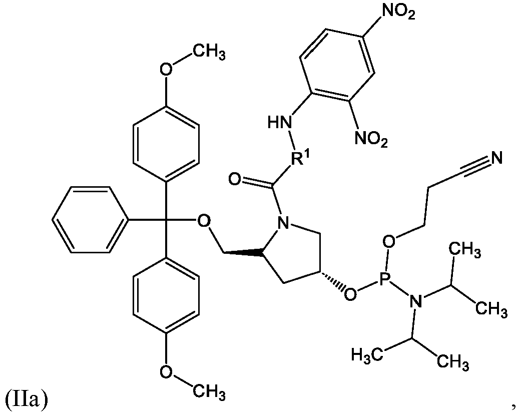 the method comprising:
contacting a compound represented by the formula: 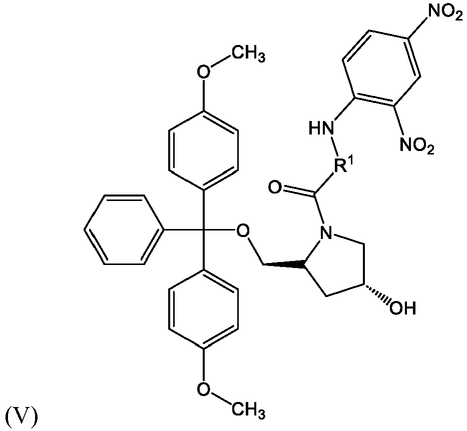 with 2-cyanoethyl N,N,N',N'-tetraisopropylphosphorodiamidite, wherein R¹ is selected from the group consisting of C₁-C₂₀-alkyl preferably C₁-C₆-alkyl, C₁-C₂₀-alkoxy preferably C₁-C₆-alkoxy, C₂-C₂₀-alkenyl preferably C₂-C₆-alkenyl, C₂-C₂₀-alkynyl preferably C₂-C₆-alkynyl, C₆-C₁₄ aryl preferably C₆ aryl, -C(=O)-NR^{a}R^{b} wherein R^{a} and R^{b} are identical or different and are C₁-C₄-alkyl optionally substituted by halogen, and -((CH₂)ₙO)ₘ wherein n is 1, 2, 3, or 4 and m is 1, 2, 3, 4, 5, 6, 7, 8, or 9,
wherein the C₁-C₂₀-alkyl, the C₁-C₂₀-alkoxy, the C₂-C₂₀-alkenyl, the C₂-C₂₀-alkynyl, the C₆-C₁₄ aryl, and the -((CH₂)ₙO)ₘ are optionally substituted, one or two or three times, identically or differently, with a halogen atom, -OH, oxo (=O), -CN, -NO₂, C₁-C₆-alkyl, C₃-C₇ cycloalkyl, C₁-C₄ dialkylamino, C₆-C₁₄ aryl optionally substituted with a halogen and 5-10-membered heteroaryl optionally substituted with a C₆-C₁₀ aryl group and optionally substituted with one to three C₁-C₄ alkyl groups.

13. A method of making a compound represented by the formula: 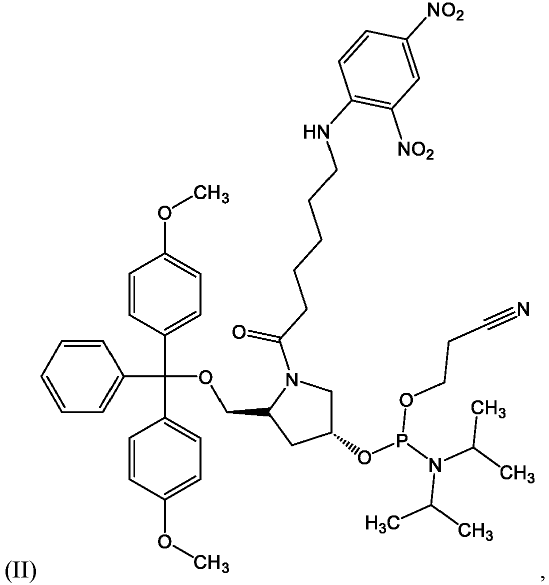 the method comprising: contacting a compound represented by the formula 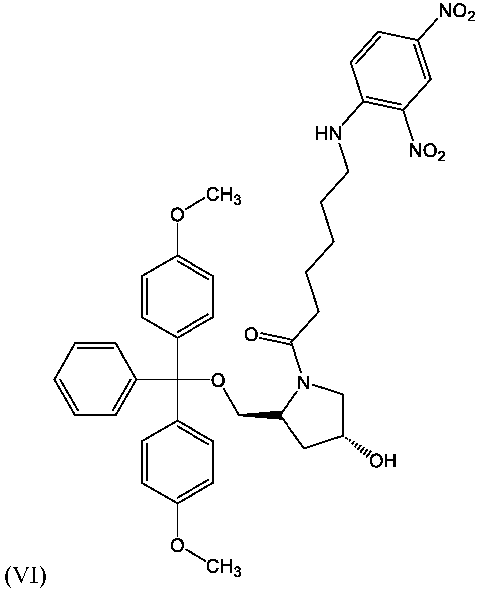 with 2-cyanoethyl N,N,N',N'-tetraisopropylphosphorodiamidite.

14. A collection of nucleic acid hybridization probes, each probe in the collection comprising a polynucleotide of claim 8 that comprises one or more hybridization segments, wherein the one or more hybridization segments are each complementary to a region in a single preselected target nucleic acid.

15. The collection of claim 14, wherein the dinitrophenyl moiety in each of the probes is located at the 5' end of the probe or at the 3' end of the probe.

16. A hybridization complex comprising the collection of probes of any one of claims 14-15 hybridized to a single preselected target nucleic acid, preferably wherein the target nucleic acid sequence is an mRNA, a gene, a non-coding RNA, a centromere, a viral RNA sequence, a viral DNA sequence, a telomere or a noncoding nucleic acid.

17. The hybridization complex of claim 16, wherein the preselected target nucleic acid sequence is a human papillomavirus (HPV) sequence, an epidermal growth factor receptor (EGFR) sequence, a ubiquitin sequence, glyceraldehyde 3 -phosphate dehydrogenase (GAPDH) sequence, a receptor tyrosine-protein kinase erbB-2 (HER2) sequence, or a Ki67 sequence.

18. A method comprising: contacting a sample comprising a target nucleic acid sequence with the collection of any one of claims 14-15 to which a detectable label has been attached directly or indirectly to the dinitrophenyl moiety and detecting the detectable label.

19. A method of detecting a target nucleic acid sequence, the method comprising contacting in vitro a biological sample comprising the target nucleic acid sequence with the collection of any one of claims 14-15 to which a detectable label has been attached directly or indirectly to the dinitrophenyl moiety and detecting the detectable label.

20. The method of any one of claims 18 or 19, wherein the presence of the detectable label indicates the presence of the target nucleic acid sequence, preferably wherein detecting comprises incubating the sample with one or more anti-DNP antibodies attached to a detectable label and, optionally, wherein detecting comprises light microscopy, fluorescence microscopy, flow cytometry, or transmission electron microscopy.

21. A method of making the collection of any one of claims 14-15, the method comprising: synthesizing the probes of the collection to generate a plurality of synthesized probes and combining the plurality of synthesized probes to generate the collection.
